# EUROPÄISCHE PATENTSCHRIFT

(11) **EP 1 667 639 B1**
(45) Veröffentlichungstag und Bekanntmachung des Hinweises auf die Patenterteilung: **30.09.2009**
(21) Anmeldenummer: 04761890.5
(22) Anmeldetag: 01.09.2004
(51) Int. Cl.: A61K 8/18

(54) **HAUTPFLEGEPRODUKT**
SKIN-CARE PRODUCT
PRODUIT DE SOINS POUR LA PEAU

(30) Priorität: 11.09.2003 CH 155103
(43) Veröffentlichungstag der Anmeldung: 14.06.2006
(73) Patentinhaber: Goloy Gmbh, CH-8610 Uster (CH)
(72) Erfinder: MARTY, Jo, CH-8706 Feldmeilen (CH)
(74) Vertreter: Vogel, Dany
(86) Internationale Anmeldenummer: PCT/CH2004/000550
(87) Internationale Veröffentlichungsnummer: WO 2005/023211

(56) Entgegenhaltungen:
- EP-A- 0 293 535
- DE-A- 1 467 783
- FR-A- 1 349 160
- GB-A- 1 077 378

## Beschreibung

Die Erfindung betrifft ein Hautpflegeprodukt, insbesondere eine Hautpflegecrème, sowie ein Verfahren zur Herstellung eines Hautpflegeprodukts.

Aus den Patentanmeldungen FR 1 354 857, GB 1 077 378 sowie EP 0 293 535 A1 sind Hautpflegeprodukte bekannt, welche ein Hydroperoxid eines Fettes enthalten. Solche Produkte weisen besondere hautpflegende und hautverändernde Eigenschaften durch ihre Fähigkeit auf, naszierenden Sauerstoff freizusetzen. DE 1 467 783 offenbart ein Hautpflegeprodukt, das eine wässrige Emulsion von Peroxiden von langkettigen ungesättigten Fetten umfasst. FR 1 349 160 offenbart eine fettfreie Schönheitslotion mit homöopathischen Zusätzen.

Es ist eine Aufgabe der vorliegenden Erfindung, hiervon ausgehend ein Hautpflegeprodukt anzugeben, welches eine verbesserte Verträglichkeit besitzt.

Diese Aufgabe wird gelöst durch ein Hautpflegeprodukt, insbesondere eine Hautpflegecrème, gemäss Anspruch 1.

Es ist eine weitere Aufgabe der Erfindung, ein einfaches Herstellungsverfahren für ein Hautpflegeprodukt gemäss Anspruch 1 anzugeben. Diese Aufgabe wird gelöst durch ein Verfahren gemäss Anspruch 6.

Vorteilhafte Ausgestaltungen der Erfindung sind in den abhängigen Ansprüchen angegeben.

Das erfindungsgemässe Hautpflegeprodukt enthält ein sogenanntes Sauerstofföl, d.h., ein Öl, das in der Lage ist, Sauerstoff freizusetzen. Chemisch handelt es sich bei dem Sauerstofföl um das Hydroperoxid eines Fettes. Das Hydroperoxid weist eine Peroxidzahl von mindestens 20 auf, bevorzugt liegt sie zwischen 40 und 100. Der Anteil des Hydroperoxides des Fettes am Hautpflegeprodukt beträgt bevorzugt zwischen 0,1 und 40 Gewichts-Prozent, insbesondere zwischen 5 und 40 Gewichts-Prozent, besonders bevorzugt zwischen 15 und 25 Gewichts-Prozent.

Eines der Ausgangsprodukte ist folglich ein Fett, welches mit geeigneten Methoden in ein Hydroperoxid überführt wird. Chemisch gesehen handelt es sich bei dem Fett vor der Überführung in das Hydroperoxid um einen Ester einer Fettsäure mit einem ein- oder mehrwertigen Alkohol, welcher vollkommen oder unvollkommen verestert sein kann. Bevorzugt handelt es sich bei dem Alkohol um den dreiwertigen Alkohol Glycerin und bei dem Fett um ein Triglycerid mit drei verschiedenen oder gleichen Fettsäureresten. Das Fett weist wenigstens einen ungesättigten Fettsäurerest auf. Bevorzugt besitzt dieser Rest zwischen 10 und 22 Kohlenstoffatome in der Fettsäurekette, und das Fett weist vorzugsweise eine Iodzahl zwischen 50 und 150 auf.

Die Peroxidzahl und die Iodzahl sind gängige Grössen zur Charakterisierung von Fetten und Ölen und werden z.B. in Römpp, Chemie-Lexikon, 9. Auflage, Thieme-Verlag Stuttgart 1990, definiert. Die Peroxidzahl ist die Menge des in 1 kg des Fettes enthaltenen aktiven Sauerstoffs (ausgedrückt in Milliequivalenten oder mEq), die in einem Gemisch aus Chloroform und Eisessig aus Kaliumiodid elementares Iod freisetzen. Die Iodzahl ist die Menge an elementarem Iod (in Gramm), welche an 100 Gramm Fett unter Aufspaltung der Doppelbindungen addiert werden. Für beide Kennziffern gibt es eine Reihe von bekannten Bestimmungsverfahren, die dem Fachmann geläufig sind, im einfachsten Fall Titrationen mit geeignetem Farbindikator. Heutzutage werden meist empfindlichere photometrische Verfahren zur Bestimmung dieser Kennzahlen eingesetzt.

Die Iodzahl ist somit ein Mass für die Zahl an verfügbaren Doppelbindungen und damit für den Gehalt an ungesättigten Fettsäuren. Diese Zahl ist insofern von Bedeutung, dass in einer Reaktion von Fetten mit molekularem Sauerstoff Hydroperoxygruppen (-O-OH) meist nahe bei Doppelbindungen in der Fettsäurekette gebildet werden.

Das Fett, dessen Hydroperoxid im erfindungsgemässen Hautpflegeprodukt enthalten ist, ist bevorzugt ein natürliches Öl oder ein Gemisch aus natürlichen Ölen, z.B. Sojaöl, Rapsöl, Sonnenblumenöl, Erdnussöl, Palmkernöl, Palmöl, Leinöl, Olivenöl, Maiskeimöl, oder Kokosöl. Vorzugsweise enthält das Fett Erdnussöl und/oder Maiskeimöl. Besonders bevorzugt wird als Fett reines Erdnussöl verwendet.

Chemisch gesehen handelt es sich bei diesen natürlichen Fetten um Triglyceride. Sie enthalten in der Regel verschiedene Fettsäuren, darunter auch ein- oder mehrfach ungesättigte Fettsäuren mit einer Kettenlänge von 10 bis 22 Kohlenstoffatomen. Einfach ungesättigte Fettsäuren in diesem Längenbereich sind z.B. Palmitoleinsäure, Ölsäure, Erucasäure. Zweifach ungesättigte Fettsäuren in diesem Bereich sind z.B. Linolsäure, dreifach ungesättigte Fettsäuren z.B. Linolensäure oder Elaeostearinsäure. Ein Beispiel für eine vierfach ungesättigte Fettsäure ist Arachidonsäure.

Die Hydroperoxide können mit jedem bekannten Verfahren zur Peroxidation von Fetten hergestellt werden. Solche Methoden sind aus dem Stand der Technik wohlbekannt und umfassen z.B. Autoxidation mit Luftsauerstoff, Oxidation mit Singlettsauerstoff oder enzymatische Reaktionen, z.B. mit Lipoxidasen. Hierbei ist es bevorzugt, dass die Reaktionsbedingungen so gewählt werden, dass andere Oxidationsprodukte in möglichst geringer Konzentration entstehen.

Ein mögliches Verfahren zur schonenden Herstellung des Hydroperoxides eines Fettes umfasst z.B. das Durchströmen des flüssigen Fettes mit einem sauerstoffhaltigen Gas, bevorzugt mit Luft. Man lässt z.B. Luft für einige Stunden bis Tage durch ein flüssiges Fett oder Öl bei einer Temperatur zwischen 20 °C und 90 °C, bevorzugt zwischen 20 °C und 70 °C, durchströmen. Dies kann bei konstanter oder veränderlicher Temperatur geschehen. In einem bevorzugten Verfahren lässt man Luft durch das Fett oder Öl bei etwa 70 °C strömen, bis die Peroxidzahl den Wert 20 erreicht. Nach dieser Zeit wird die Temperatur auf etwa 40 °C gesenkt, und es wird weiterhin Luft durch das Fett oder Öl hindurchgeströmt, bis die Peroxidzahl einen vorgegebenen Endwert erreicht. Durch diese Verfahren wird ein Hydroperoxid mit einer sehr geringen Konzentration an anderen Autoxidationsprodukten erreicht.

Die Hydroperoxide von Fetten, insbesondere die auf die soeben beschriebene Art hergestellten Hydroperoxide, haben die bemerkenswerte Eigenschaft, sehr stabile Emulsionen mit Wasser zu bilden. Die Herstellung eines Hautpflegeproduktes in Form einer Creme oder Lotion, die ein Hydroperoxid enthält, kann daher sehr einfach in zwei Schritten erfolgen. Zunächst wird eine Grundemulsion eines beliebigen geeigneten Öls mit Wasser hergestellt. Der Emulsionstyp ist bevorzugt Öl-in-Wasser. Jedes bekannte Emulgationsverfahren kann hierzu verwendet werden, z.B. Durchpressen durch eine Homogenisierdüse unter Druck. Danach wird das Fett-Hydroperoxid hinzugefügt. Dieses lässt sich leicht in die bestehende Emulsion hinein emulgieren, insbesondere durch Rühren. Auf diese Weise können selbst grosse Mengen des Fett-Hydroperoxids der Grundemulsion zugefügt werden, ohne dass die Emulsion ihre Stabilität verliert.

Der pH-Wert des Hautpflegeprodukts liegt bevorzugt zwischen 7 und 9, besonders bevorzugt zwischen 8 und 9, da sich in diesem Bereich die Stabilität der Emulsion als am höchsten erwiesen hat. Des weiteren wird in diesem pH-Bereich eine gute Hautverträglichkeit gewährleistet. Der pH-Wert kann auf jede bekannte Weise eingestellt werden.

Im Zusammenhang mit Fett-Hydroperoxiden, ihren Eigenschaften und Anwendungsmöglichkeiten wird weiterhin auf das Dokument GB 1 077 378 von P. Baranger verwiesen.

Ein weiterer essentieller Bestandteil des erfindungsgemässen Hautpflegeprodukts ist mindestens eine Substanz, welche aus einer Gruppe ausgewählt ist, die aus den Substanzen Kaliumchlorid, Calciumfluorid und Kieselsäure besteht, bevorzugt in einem Anteil von mindestens 5·10⁻¹² Gewichts-Prozent, besonders bevorzugt von mindestens 5·10⁻⁷ Gewichts-Prozent. Insbesondere liegt der Anteil dieser Substanz bevorzugt zwischen 5·10⁻¹² und 5·10⁻³ Gewichts-Prozent am Gesamtprodukt. Diese Substanz wird im folgenden auch als Mineralstoffkomponente bezeichnet. Unter Kieselsäure ist in diesem Zusammenhang jede Verbindung der allgemeinen Formel SiO₂ · n H₂O mit beliebigem ganz- oder nichtganzzahligem Wert von n zu verstehen, insbesondere Orthokieselsäure, Pyrokieselsäure, Polykieselsäuren oder polymeres Siliciumdioxid (Kieselsäureanhydrid). Die Angabe des Gewichtsanteils bezieht sich hierbei auf den Gehalt an Kieselsäure umgerechnet auf das Kieselsäureanhydrid, also den Gehalt an SiO₂ im Kieselsäureanteil des Produktes.

Vorzugsweise enthält das Hautpflegeprodukt alle drei Substanzen (Mineralstoffkomponenten), bevorzugt jeweils in einem Anteil zwischen 5·10⁻¹² und 5·10⁻³ Gewichts-Prozent, besonders bevorzugt, zwischen 5·10⁻⁷ und 5·10⁻⁵ Gewichts-Prozent.

Diese Mineralstoffkomponenten sind aus der klassischen Homöopathie bekannt und werden dort als Kalium chloratum, Calcium fluoratum und Silicea bezeichnet.

Kalium chloratum werden in geeigneter Konzentration (in der Sprache der Homöopathie auch Potenzierung genannt) allgemein beruhigende, gefässstabilisierende Wirkungen zugeschrieben. Calcium fluoratum werden stabilisierende Wirkungen auf die strukturellen Proteine wie Keratin oder Elastin zugeschrieben; somit trägt es zur Flexibilität und Festigkeit der daraus gebildeten Strukturen bei. Silicea schliesslich hat positive Wirkungen auf das Bindegewebe.

Die Konzentration homöopathischer Wirkstoffe wird meist als Potenzierung in der D-Skala angegeben. Hierbei bedeutet z.B. eine Potenzierung von D3, dass der Wirkstoff in 10³-facher Verdünnung, also in einer Konzentration von 10⁻³ bezogen auf das Gewicht, letztlich also 0,1 Gewichts-Prozent, vorliegt. Entsprechend bedeutet D6 eine Konzentration von 10⁻⁶ Gewicht/Gewicht, also 10⁻⁴ Gewichts-Prozent, usw.

In Versuchen mit Probanden zeigte sich nun überraschend, dass ein Hautpflegeprodukt, welches neben einem Hydroperoxid eines Fettes auch noch mindestens eine der genannten Mineralstoffkomponenten in geringer Konzentration enthält, eine weitaus verbesserte Pflegewirkung und Verträglichkeit zeigt. Die genannten Verbesserungen sind unerwartet und weitaus grösser, als dies aufgrund der Eigenschaften der jeweiligen Komponenten (Fett-Hydroperoxid bzw. Mineralstoffkomponente) alleine zu erwarten wäre. Der grösste Effekt ergibt sich, wenn alle drei Mineralstoffkomponenten Kaliumchlorid, Calciumfluorid und Kieselsäure gemeinsam anwesend sind. Überraschenderweise zeigen schon sehr kleine Mengen der Mineralstoffkomponenten die erfindungsgemässe Wirkung.

Der genaue Wirkmechanismus ist zur Zeit noch nicht bekannt. Offenbar liegt jedoch ein synergistischer Effekt im Zusammenwirken der Komponenten vor. So ist einerseits bekannt, dass Fett-Hydroperoxide schon alleine den Sauerstoffgehalt in den Hautzellen erhöhen können. Andererseits ist bekannt, dass die körpereigenen Mineralstoffe, insbesondere Kalium- und Calciumionen, eine essentielle Rolle in der Signalübertragung zwischen Körperzellen und im Stoffwechsel der Zellen spielen. Hierbei liegen diese Ionen häufig in äusserst geringen Konzentrationen vor. Möglicherweise beschleunigen in dem erfindungsgemässen Produkt die Mineralstoffkomponenten die Aufnahme der anderen Cremebestandteile in die Hautzellen und ihre Wirkung auf den Stoffwechsel, insbesondere die Aufnahme der Fett-Hydroperoxide und die Freisetzung von Sauerstoff im Inneren der Zellen. Auch ist es denkbar, dass das Zusammenspiel der Fett-Hydroperoxide mit den Mineralstoffkomponenten die Signalübertragung in den Zellrezeptoren positiv beeinflusst. Die Komplexität der biochemischen Vorgänge in den Hautzellen bedingt dabei eine gewisse Wahrscheinlichkeit, dass der genaue Wirkmechanismus ebenfalls komplex sein wird. Ausser Zweifel steht jedoch die empirisch erwiesene unerwartete Wirkung des erfindungsgemässen Hautpflegeprodukts.

In einem vorteilhaften Herstellungsverfahren werden die Mineralstoffkomponenten in wässriger Lösung bzw. Suspension in einer Potenzierung zwischen D3 und D12, bevorzugt D5 bis D7, besonders bevorzugt D6, zu einer-Grundemulsion zugegeben, der schon ein Fett-Hydroperoxid zugefügt sein kann. Alternativ kann das Fett-Hydroperoxid auch nach den Mineralstoffkomponenten zugegeben werden. Die Menge wird so gewählt, dass der Anteil der Mineralstoff-Lösung am Endprodukt 2 bis 10 Gewichts-Prozent, bevorzugt etwa 5 Gewichts-Prozent, beträgt. Damit ergibt sich ein bevorzugter gesamter Anteil jeder Mineralstoffkomponente im Endprodukt zwischen 5·10⁻¹² und 5·10⁻³ Gewichts-Prozent, bevorzugt 5·10⁻⁷ und 5·10⁻⁵ Gewichts-Prozent, besonders bevorzugt etwa 5·10⁻⁶ Gewichts-Prozent. Während im bevorzugten Konzentrationsbereich eine optimale Wirkung beobachtet wird, ist in dem gesamten angegebenen Bereich eine besondere Wirkung zu erwarten.

Die Wirkung lässt sich weiter dadurch verbessern, dass das Hautpflegeprodukt ein elektromagnetisch behandeltes Wasser in einer Konzentration zwischen 0,1 und 30 Gewichts-Prozent, bevorzugt zwischen 0,5 und 4 Gewichts-Prozent, enthält. Das Wasser wird mit einem sogenannten radionischen Kopiergerät behandelt. Solche Geräte sind im Fachhandel erhältlich und wohlbekannt. Ein früher häufig eingesetztes Kopiergerät war das Modell MC von Bruce Copen. Heute sind radionische Kopiergeräte z.B. über Copen Labs, Radionic Research Services and Supplies, 35 Beaufort Court, Admirals Way, South Quai Waterside, London, E149XL, England erhältlich.

Zur Herstellung des elektromagnetisch behandelten Wassers wird in einem Zielbehälter im radionischen Kopiergerät zunächst reines Quellwasser bereitgestellt. In einem Behälter für die Ausgangssubstanz wird Alkohol bereitgestellt. Mit dem radionischen Kopiergerät wird das Signal des Alkohols auf das Quellwasser übertragen. Anschliessend wird im Behälter für die Ausgangssubstanz ein Wasser aus einer hochgelegenen Quelle, bevorzugt einer hochgelegenen Himalaya-Quelle, eingefüllt und dessen Signal auf das Quellwasser übertragen. Das so hergestellte Wasser unterscheidet sich nach heutigen Erkenntnissen physiko-chemisch nicht von normalem, reinem Quellwasser. Jedoch trägt es das strukturelle Molekularmuster, so dass die ursprüngliche biophysikalische Molekularoszillation auf das Wasser übertragen wird. Das heisst, der Träger Wasser übernimmt als Reaktion auf die piezonelektrischen Impulse sowohl das Ladungsmuster wie die Vektorisierung der vorhandenen Kationen und Anionen (im Sinne einer Feld-Kraft-Übertragungs-Resonanz). Das beschriebene Phänomen ist mit heutigen physiko-chemischen Methoden nicht nachweisbar, es weist jedoch empirisch positive Wirkungen auf den Menschen auf. Die hier vermittelte Information ist Information über das Wasser einer hochgelegenen Wasserquelle im Himalaya, welches in verschiedensten Versuchen aussergewöhnliche Eigenschaften zeigte. Diese Information wird im Wasser mit Hilfe der zuvor übertragenen Alkohol-Information fixiert.

Das erfindungsgemässe Hautpflegeprodukt liegt bevorzugt als Creme vor, kann aber auch als Lotion, Gel oder in anderer geeigneter Form vorliegen. Es eignet sich für eine Anwendung vor allem im Gesicht, kann aber auch für die Pflege der Hände, anderer Extremitäten oder anderer Körperstellen eingesetzt werden. Es kann insbesondere zur Vorbeugung und/oder Behandlung von Alterungserscheinungen der Haut dienen, besonders solcher Alterungserscheinungen, die auf eine verminderte Sauerstoffversorgung zurückzuführen sind.

### Beispiel

Eine Hautcreme wurde wie folgt hergestellt, wobei alle Prozentangaben den Gewichtsanteil bezogen auf das Endprodukt angeben: Eine Grundemulsion wurde aus destilliertem Wasser und einer Mischung aus natürlichen Ölen mit Glycerylstearat und Ethyllinoleat als Emulgatoren hergestellt. Weiterhin wurden 20% eines Hydroperoxides von Erdnussöl, 5% einer Mineralsalzlösung, enthaltend Kaliumchlorid, Calciumfluorid und Kieselsäure in einer Konzentration von jeweils 10⁻⁴ Gewichts-Prozent (also in Potenzierung D6), sowie 2% des mit der Information einer Himalaya-Quelle elektromagnetisch behandelten Wassers zugegeben und eingerührt. Des weiteren wurden weitere übliche Inhaltsstoffe von handelsüblichen Gesichtscrèmes zugesetzt, insbesondere Glycerin, Aloe-Vera-Extrakt, Avocadoöl, Jojobaöl, Vitamin A, und Parfumstoffe. Die Inhaltsstoffe gemäss INCI-Norm (International Nomenclature of Cosmetic Ingredients) lauten: Natural Oil, Aqua, Glycerin, Aloe Barbadensis, Persia Gratissima, Buxus sinensis, Caprylic/Capric Triglyceride, Glyceryl Stearate SE, Pentylene Glycol, Octyl Octanoate, Ethyl Linoleate, Retinol, Trilinolein.

Es wurde eine Feldstudie an 200 freiwilligen Testpersonen (männlich und weiblich) über einen Zeitraum von zwei Monaten durchgeführt. Die Probanden erhielten die Hautcreme zur Anwendung gemäss ihren üblichen Pflegegewohnheiten. Die Feldprobanden waren alle älter als 20 Jahre und wendeten die Produkte regelmässig während der Dauer der Testphase als Tages- und Nachtpflege an. Mittels eines Fragebogens teilten anschliessend die Probanden ihre Eindrücke zum Produkt und dessen Wirksamkeit mit. Spezielles Gewicht wurde im Fragebogen auf die Fragen der Wirkung und der Veränderung des Hautbildes sowie auf die Verträglichkeit gelegt. Ebenfalls wurden Hauttypologien, Hautallergien und Hautreaktionen sowie spezielle sichtbare Veränderungsmerkmale (z.B. Augen-, Mund- und Decolleté-Partien) während der Testphase erfasst.

Die Studie ergab eine überaus gute Verträglichkeit und höhere Wirksamkeit gegenüber den von den Probanden üblicherweise verwendeten Vergleichscrèmes. So führte die Crème insbesondere schon bald nach dem Auftrag zu einer deutlichen Entspannungswirkung in der Haut der Probanden. Selbst nach mehrwöchigem täglichem Auftrag wurden keine Unverträglichkeitsreaktionen beobachtet.

Im Gegenteil, die Probanden stellten fest, dass Unreinheiten der Haut nach kurzer Zeit verschwanden, Rötungen im speziellen im Bereich der Nase und der Augenpartien sich verminderten, die Haut straffer und geschmeidiger wurde, da die Durchblutung sich verbesserte. Selbst bei Allergikern war die Verträglichkeit gut und es traten auch nach längerer Anwendung keine negativen Reaktionen auf.

Weiterhin wurden physiko-chemische Tests sowie verschiedene Untersuchungen auf der Basis der Hautzellmembran-Potenzialdifferenz durchgeführt. Unter anderem wurden Messungen der optischen Aktivität mit Hilfe von Linear-Polarisationsfiltern durchgeführt. Des weiteren wurden Kenngrössen der biophysikalischen Molekularoszillation ermittelt, insbesondere die radiästhetische Intensität mithilfe eines sogenannten aperiodischen Resonators, dessen Schwingungsenergie, das Ausschwingverhalten eines schwingenden Kondensators, und die Resonanzfrequenzen mit ihren Polaritäten. Weiterhin wurden eine Überprüfung mit Farbtafeln 230, Halbwellenmessungen und Feldstärkemessungen mit bifilaren Messingspiralen vorgenommen. Schliesslich wurde bei einem Probanden die Augengrenzfrequenz mit Hilfe der Flimmer-Verschmelzungs-Photometrie nach Petöfalvi ermittelt. Alle diese Untersuchungen zeigten, dass auch mit den Massstäben der genannten biophysikalischen Methoden das erfindungsgemässe Hautpflegeprodukt sehr aussergewöhnliche Eigenschaften aufweist.

Dies wurde ebenfalls bei den mikrobiologischen Untersuchen bestätigt. Die Untersuchungen wurden gemäss CTFA - microbiological limit guideline for cosmetics and toileteries (1985) - und CTPA - microbial quality management, limits and guidelines (1990) - durch ein unabhängiges Labor durchgeführt.

## Patentansprüche

1. Hautpflegeprodukt, insbesondere Hautpflegecrème, enthaltend ein Hydroperoxid eines Fettes, wobei das Hydroperoxid eine Peroxidzahl von mindestens 20 aufweist, **dadurch gekennzeichnet, dass** das Hautpflegeprodukt mindestens eine Substanz ausgewählt aus der Gruppe bestehend aus Kaliumchlorid, Calciumfluorid und Kieselsäure enthält, wobei der Anteil der mindestens einen Substanz am Hautpflegeprodukt zwischen 5·10⁻¹² und 5·10⁻³ Gewichts-Prozent beträgt, und dass der Anteil des Hydroperoxids des Fettes am Hautpflegeprodukt zwischen 0,1 und 40 Gewichts-Prozent beträgt.

2. Hautpflegeprodukt gemäss Anspruch 1, **dadurch gekennzeichnet, dass** es alle drei Substanzen der Gruppe bestehend aus Kaliumchlorid, Calciumfluorid und Kieselsäure enthält, wobei jede der Substanzen einen Anteil am Hautpflegeprodukt zwischen 5·10⁻¹² und 5·10⁻³ Gewichts-Prozent aufweist.

3. Hautpflegeprodukt gemäss Anspruch 2, **dadurch gekennzeichnet, dass** der Anteil von Kaliumchlorid, Calciumfluorid und Kieselsäure jeweils zwischen 5·10⁻⁷ und 5·10⁻⁵ Gewichts-Prozent liegt.

4. Hautpflegeprodukt gemäss einem der Ansprüche 1 bis 3, **dadurch gekennzeichnet, dass** es weiterhin ein in einem radionischen Kopiergerät elektromagnetisch behandeltes Wasser in einem Anteil zwischen 0,1 und 30 Gewichts-Prozent enthält.

5. Hautpflegeprodukt gemäss einem der Ansprüche 1 bis 4, **dadurch gekennzeichnet, dass** das Fett, dessen Hydroperoxid im Hautpflegeprodukt enthalten ist, Erdnussöl ist.

6. Verfahren zur Herstellung eines Hautpflegeproduktes gemäss Anspruch 1, umfassend die Schritte
- Herstellen einer Grundemulsion enthaltend Wasser und wenigstens ein natürliches Öl;
- Hinzufügen einer wässrigen Lösung oder Suspension von wenigstens einer der Substanzen Kaliumchlorid, Calciumfluorid und Kieselsäure, so dass der Anteil der mindestens einen Substanz am Hautpflegeprodukt zwischen 5·10⁻¹² und 5·10⁻³ Gewichts-Prozent beträgt;
- Hinzufügen eines Hydroperoxides eines natürlichen Fettes, wobei das Hydroperoxid eine Peroxidzahl von mindestens 20 aufweist und wobei der Anteil des Hydroperoxids am Hautpflegeprodukt zwischen 0,1 und 40 Gewichts-Prozent beträgt und; und
- Emulgieren des resultierenden Gemisches, um eine homogene Emulsion zu erzielen.

7. Verfahren gemäss Anspruch 6, **dadurch gekennzeichnet, dass** es vor dem Schritt des Hinzufügens des Hydroperoxides weiterhin den Schritt umfasst
- Herstellen eines Hydroperoxides eines natürlichen Fettes, indem das natürliche Fett mit einem sauerstoffhaltigen Gas durchströmt wird, bis das **dadurch** veränderte Fett eine Peroxidzahl von mindestens 20 aufweist.

8. Verfahren gemäss Anspruch 6 oder 7, **dadurch gekennzeichnet, dass** es weiterhin die Schritte umfasst
- Bereitstellen von Wasser in einem Zielbehälter eines radionischen Kopiergeräts;
- Bereitstellen von Wasser einer Wasserquelle des Himalaya in einem Ausgangsbehälter eines radionischen Kopiergeräts;
- Überragen von Information vom Ausgangsbehälter zum Zielbehälter mit Hilfe des radionischen Kopiergeräts;
- Zufügen des Wassers im Zielbehälter zur Grundemulsion.

## Claims

1. A skin care product, in particular a skin care cream, containing a hydroperoxide of a fat, wherein the hydroperoxide has a peroxide number of at least 20, **characterized in that** the skin care product contains at least one substance selected from the group consisting of potassium chloride, calcium fluoride and silicic acid, wherein the proportion of the at least one substance in the skin care product is between 5 × 10⁻¹² and 5 × 10⁻³ percent by weight, and **in that** the proportion of fat hydroperoxide in the skin care product is between 0.1 and 40 percent by weight.

2. The skin care product according to claim 1, **characterized in that** it contains all three substances of the group consisting of potassium chloride, calcium fluoride and silicic acid, wherein the proportion of each of the substances in the skin care product is between 5 × 10⁻¹² and 5 × 10⁻³ percent by weight.

3. The skin care product according to claim 2, **characterized in that** the proportion of potassium chloride, calcium fluoride and silicic acid is each between 5 × 10⁻⁷ and 5 × 10⁻⁵ percent by weight.

4. The skin care product according to one of claims 1 to 3, **characterized in that** it further contains water, which has been treated electromagnetically in a radionic copier, in a proportion of between 0.1 and 30 percent by weight.

5. The skin care product according to one of claims 1 to 4, **characterized in that** the fat contained as the hydroperoxide in the skin care product is peanut oil.

6. A method for manufacturing a skin care product according to claim 1, comprising the following steps:
- producing a base emulsion containing water and at least one natural oil;
- adding an aqueous solution or suspension of at least one of the substances potassium chloride, calcium fluoride and silicic acid, so that the proportion of the at least one substance in the skin care product is between 5 × 10⁻¹² and 5 × 10⁻³ percent by weight;
- adding a hydroperoxide of a natural fat, wherein the hydroperoxide has a peroxide number of at least 20 and wherein the proportion of the hydroperoxide in the skin care product is between 0.1 and 40 percent by weight; and
- emulsifying the resulting mixture to produce a homogeneous emulsion.

7. The method according to claim 6, **characterized in that** prior to the step for adding the hydroperoxide, it comprises the following step:
- producing a hydroperoxide of a natural fat, wherein the natural fat is flushed with an oxygen-containing gas until the altered fat has a peroxide number of at least 20.

8. The method according to claim 6 or claim 7, **characterized in that** it further comprises the following steps:
- placing water in a target container of a radionic copier;
- placing water from a Himalayan water source in a starting container of a radionic copier;
- transferring information from the starting container to the target container with the aid of the radionic copier;
- adding the water in the target container to the base emulsion.

## Revendications

1. Produit de soin pour la peau, notamment crème de soin pour la peau, contenant un hydroperoxyde de graisse, l'hydroperoxyde présentant un indice de peroxyde d'au moins 20, **caractérisé en ce que** le produit de soin pour la peau contient au moins une substance sélectionnée parmi le groupe composé du chlorure de potassium, du fluorure de calcium et de l'acide silicique, la teneur de l'au moins une substance en produit de soin pour la peau étant comprise entre 5.10⁻¹² et 5.10⁻³ pour cent en poids et la teneur de l'hydroperoxyde de graisse en produit de soin pour la peau étant comprise entre 0,1 et 40 pour cent en poids.

2. Produit de soin pour la peau selon la revendication 1, **caractérisé en ce qu'**il contient les trois substances du groupe composé du chlorure de potassium, du fluorure de calcium et de l'acide silicique, chacune des substances présentant une teneur en produit de soin pour la peau comprise entre 5.10⁻¹² et 5.10⁻³ pour cent en poids.

3. Produit de soin pour la peau selon la revendication 2, **caractérisé en ce que** la teneur en chlorure de potassium, fluorure de calcium et acide silicique est respectivement comprise entre 5.10⁻⁷ et 5.10⁻⁵ pour cent en poids.

4. Produit de soin pour la peau selon une des revendications 1 à 3, **caractérisé en ce qu'**il contient en outre une eau traitée par électromagnétisme dans un copieur radionique en une teneur comprise entre 0,1 et 30 pour cent en poids.

5. Produit de soin pour la peau selon une des revendications 1 à 4, **caractérisé en ce que** la graisse dont l'hydroperoxyde est contenu dans le produit de soin pour la peau est de l'huile d'arachide.

6. Procédé de préparation d'un produit de soin pour la peau selon la revendication 1, comprenant les étapes de
- préparation d'une émulsion de base contenant de l'eau et au moins une huile naturelle ;
- ajout d'une solution aqueuse ou d'une suspension d'au moins une des substances chlorure de potassium, fluorure de calcium et acide silicique de manière à ce que la teneur de l'au moins une substance en produit de soin pour la peau soit comprise entre 5.10⁻¹² et 5.10⁻³ pour cent en poids ;
- ajout d'un hydroperoxyde de graisse naturelle, l'hydroperoxyde présentant un indice de peroxyde d'au moins 20 et la teneur de l'hydroperoxyde de graisse en produit de soin pour la peau étant comprise entre 0,1 et 40 pour cent en poids ; et
- émulsification du mélange résultant afin d'obtenir une émulsion homogène.

7. Procédé selon la revendication 6, **caractérisé en ce que**, avant l'étape d'ajout de l'hydroperoxyde, il comprend en outre l'étape de
- préparation d'un hydroperoxyde de graisse naturelle en effluvant la graisse naturelle avec un gaz contenant de l'oxygène jusqu'à ce que la graisse ainsi modifiée présente un indice de peroxyde d'au moins 20.

8. Procédé selon la revendication 6 ou 7, **caractérisé en ce qu'**il comprend aussi les étapes de
- versage d'eau dans un récipient de destination d'un copieur radionique ;
- versage d'eau d'une source d'eau de l'Himalaya dans un récipient de départ d'un copieur radionique ;
- transfert d'informations du récipient de départ au récipient de destination à l'aide du copieur radionique ;
- ajout de l'eau dans le récipient de destination destiné à l'émulsion de base.
